# EUROPÄISCHE PATENTSCHRIFT

(11) **EP 3 654 932 B1**
(45) Veröffentlichungstag und Bekanntmachung des Hinweises auf die Patenterteilung: **20.10.2021**
(21) Anmeldenummer: 18740140.1
(22) Anmeldetag: 03.07.2018
(51) Int. Cl.: A61K 8/42, A61K 8/64, A61Q 15/00, A61K 8/99, A41B 11/00

(54) **TYROTHRICIN ZUR ANWENDUNG BEI DER BEHANDLUNG ODER PROPHYLAXE VON KÖRPERGERUCH SOWIE ZUBEREITUNGEN HIERFÜR**
TYROTHRICIN FOR USE IN THE TREATMENT OR PROPHYLAXIS OF BODY ODOUR AND PREPARATIONS THEREFOR
TYROTHRICINE DESTINÉE À ÊTRE UTILISÉE LORS DU TRAITEMENT OU DE LA PROPHYLAXIE DE L'ODEUR CORPORELLE AINSI QUE PRÉPARATIONS CORRESPONDANTES

(30) Priorität: 20.07.2017 DE 102017116407
(43) Veröffentlichungstag der Anmeldung: 27.05.2020
(73) Patentinhaber: Leibbrand, Thomas, 74074 Heilbronn (DE)
(72) Erfinder: Leibbrand, Thomas, 74074 Heilbronn (DE)
(74) Vertreter: Eisenführ Speiser
(86) Internationale Anmeldenummer: PCT/EP2018/067991
(87) Internationale Veröffentlichungsnummer: WO 2019/015955

(56) Entgegenhaltungen:
- CN-U- 205 682 452
- US-A- 3 624 200
- US-A- 5 707 610
- US-A1- 2016 081 916

## Beschreibung

Die vorliegende Erfindung betrifft die therapeutische oder nicht-therapeutische Verwendung von Tyrothricin zur Behandlung und/oder Prophylaxe von Körpergeruch bei Menschen, vorzugsweise durch lokale (topische), oberflächliche Anwendung und/oder vorzugsweise durch Anwendung auf der wundfreien Haut und/oder Schleimhaut. Weiter betrifft die Erfindung eine therapeutische, vorzugsweise pharmazeutische, oder nicht-therapeutische, vorzugsweise kosmetische, Zusammensetzung, enthaltend Tyrothricin und ein Keratolytikum, vorzugsweise zur Behandlung und/oder Prophylaxe von Körpergeruch bei Menschen. Ebenfalls betrifft die Erfindung ein Kit, enthaltend Tyrothricin und ein Keratolytikum.

Körpergeruch, insbesondere als unangenehm empfundener Körpergeruch beim Menschen kann vor allem ausgehen von der Hautoberfläche, insbesondere vom Fuß, der Achselhöhle oder von schlecht durchlüfteten Hautfalten ("intertriginösen Bereichen"), etwa in der Leistengegend (inguinal) oder unter dem Brustbereich (submammär), oder kann auch ausgehen von Schleimhäuten (Mucosa), etwa der Mundschleimhaut oder der Vaginalschleimhaut. Als unangenehm empfundener Körpergeruch der vorgenannten Art bzw. Herkunft wird nach heutiger Kenntnis in aller Regel hervorgerufen und/oder gefördert und/oder ausgelöst durch bakterielle Besiedlung und/oder gewisse bakterielle Stoffwechselprodukte.

Im Fall der Herkunft von der Hautoberfläche wird als unangenehm empfundener Körpergeruch vor allem durch Schweißbildung, insbesondere durch vermehrte oder übermäßige Schweißbildung, begünstigt bzw. gefördert. Offenbar spielen Stoffwechselprodukte einiger, die Hautoberfläche besiedelnden Mikroorganismen, vor allem Bakterien, welche Hautbestandteile und andere, mit dem Schweiß abgegebene Substanzen zersetzen, eine wichtige Rolle bei der Entstehung dieses Körpergeruchs. Auch scheint die genaue Zusammensetzung und Menge der bakteriellen Besiedlung von Hautpartien einen Einfluss auf die Art und das Ausmaß der Geruchsentwicklung zu haben. Von frisch gebildetem, unzersetztem Schweiß geht dagegen kein oder nur geringer Geruch aus.

Begünstigt oder gefördert wird die unerwünschte Geruchsbildung an der Hautoberfläche durch Einflussfaktoren wie starkes bis übermäßiges Schwitzen und schlecht durchlüftete Bekleidung. Übermäßiges Schwitzen ist auch unter dem Begriff "Hyperhidrose", insbesondere unter dem Begriff "primäre fokale Hyperhidrose" bekannt. Übermäßiges Schwitzen im Fußbereich ist auch unter den Begriffen "*Hyperhidrosis plantaris*" sowie "*Hyperhydrosis pedis*/*pedum*" bekannt. Übermäßiges Schwitzen im Bereich der Achselhöhle ist auch unter dem Begriff "*Hyperhidrosis axillaris*" bekannt.

Übermäßiges Schwitzen geht oft mit einer Erscheinung einher, welche als "Bromhidrose" bekannt ist: Bromhidrose bezeichnet übelriechenden Schweiß und ist eine Sonderform der Hyperhidrose, bei welcher der vermehrt produzierte Schweiß die Hornschicht der Haut ständig durchfeuchtet und die Vermehrung der dort ansässigen Keimflora begünstigt. Mit dem Abbau des Keratins der Hornhaut entstehen Abbauprodukte, z.B. kurzkettige Fettsäuren und Amine, welche durch bestimmte Keime besagter Keimflora zu verschiedenen Sekundärprodukten weiter verstoffwechselt werden können. Einige dieser Keime scheinen über eine Vielzahl von Stoffwechselwegen zu verfügen, die zum Teil hinsichtlich des Anfalls bestimmter Stoffwechselprodukte außergewöhnlich sind: eine Besonderheit ist beispielsweise die bei einigen Mikroorganismen beobachtete Synthese von Mycothiol anstelle von Glutathion. Weiter scheinen auch flüchtige (vor allem kurzkettige) organische Thiole als Stoffwechselprodukte einiger der Keime besagter Keimflora zu entstehen. Flüchtige organische Thiole haben oft einen von Menschen als besonders unangenehm, teils als widerwärtig, empfundenen Geruch und können daher erheblich zu einem als unangenehm empfundenen Körpergeruch beitragen - vor allem im Bereich der Leistenregion, der Achselhöhlen und der Füße, etwa der Zehenzwischenräume.

Aus den Duftschweißdrüsen stammender apokriner Schweiß oder Talg wird durch Bakterien abgebaut, was zu starkem Geruch führt, der je nach Zusammensetzung der Abbau- bzw. Stoffwechselprodukte variieren kann. Unter "apokriner Sekretion" wird allgemein der Vorgang verstanden, wenn Sekretvesikel mit umgebendem apikalem Zytoplasma durch einen Teil der Zellmembran von der Drüsenzelle abgeschnürt werden.

Die Erscheinung der Bromhidrose kann noch weiter differenziert werden in die Unterformen "apokrine Bromhidrose" und "ekkrine Bromhidrose". Ekkrine Drüsen sind Drüsen, welche ihre Absonderung, das Sekret, an eine freie Oberfläche (z.B. die Haut oder den Verdauungstrakt) abgeben, wobei die Sekretbildung ohne lichtmikroskopisch erkennbaren Zytoplasmaverlust bei der Absonderung erfolgt (z.B. kleine Schweißdrüsen).

Die apokrine Bromhidrose bezeichnet eine ausgeprägte Sekretion von Schweiß, vorwiegend durch apokrine axilläre Schweißdrüsen. Organische Bestandteile des apokrinen Schweißes werden dann durch Bakterien zu unangenehm riechenden Stoffwechselprodukten zersetzt. Mangelnde Hygiene begünstigt das Auftreten apokriner Bromhidrose. Sie tritt vorwiegend im geschlechtsreifen Alter auf.

Ekkrine Bromhidrose bezeichnet unangenehmen Geruch durch Sekretion ekkrinen Schweißes unterschiedlicher Ursache. Der Unterfall der keratogenen ekkrinen Bromhidrose bezeichnet einen Zustand, wobei eine Hyperhidrose, bis hin zur exzessiven Hyperhidrose, zu einer Aufweichung der obersten Hautschicht (oberste Schicht der Epidermis; *Stratum corneum*) führt, welche dann von Bakterien zersetzt wird, mit darauffolgender Geruchsbildung. Diese Erscheinung ist auch als "Bromhyperhidrose" bekannt. Die keratogene ekkrine Bromhidrose tritt vor allem im jungen und mittleren Erwachsenenalter auf, gelegentlich auch bei Kindern, insbesondere männlichen Geschlechts. Betroffen sind vor allem die Bereiche der Fußsohlen (plantar), der Handflächen (palmar) sowie intertriginöse Bereiche.

Als unangenehm empfundener Körpergeruch, welcher vom Fuß ausgeht, wird u.a. durch schlecht durchlüftete Fußbekleidung begünstigt. So finden sich nach längerem Tragen von engem und schlecht durchlüftetem Schuhwerk oft an der durch die angestaute Feuchtigkeit aufgequollenen und teilweise mazerierten Leistenhaut - vor allem der stärker belasteten Hautpartien - grübchenförmige Hornhautläsionen, die in der Lupenvergrößerung wie ein oberflächlicher Lochfrass oder ausgestanzt und kraterförmig erscheinen. Diese Hautläsionen werden auch als "pits" bezeichnet. Diese Befunde sind auch als '*Keratoma sulcatum*', "*pitted keratolysis*" oder "*plantar pitting*" bekannt. Das Abtragen der oberflächlichen Hornhautschichten, z.B. mittels handelsüblicher Raspeln kann unangenehmen, vom Fuß ausgehenden, Körpergeruch zwar zeitweise vermindern, jedoch in der Regel nicht ganz beseitigen. Die vorstehend beschriebenen "pits" treten immer wieder auf.

Von den Betroffenen kann ihr auf die vorgenannte Weise entstandener eigener Körpergeruch als stark belastend empfunden werden, bis hin zur Ausbildung psychischer Probleme. Obwohl Betroffene sich oft mehrmals am Tag waschen, bleibt die Vorstellung "unangenehm zu riechen", was sich in extremen Fällen bis zu Geruchswahnvorstellungen als besonderer Form einer Dysmorphophobie steigern kann.

Zur Behandlung von als unangenehm empfundenem Körpergeruch, vor allem des vom Fuß oder der Achselhöhle ausgehenden Körpergeruches, sind bereits verschiedene Mittel bzw. Verfahren vorgeschlagen worden. Zu diesen zählen, neben der Verbesserung der Hygiene und der Verwendung gut durchlüfteter Bekleidung, antibakterielle Seifen oder Lotionen zur Wäsche der betroffenen Körperteile (oft z.B. enthaltend den Wirkstoff Triclosan); regelmäßige Fußbäder mit z.B. Teebaumöl; Deodorants oder Anti-Transpirantien, z.B. enthaltend Aluminiumsalze wie Aluminiumtrichlorid; Anwendung von meist schweißtrocknenden Fußcremes oder Pudern, z.B. enthaltend Zinkoxid, Zink und/oder Duftstoffe; Duftstoffe enthaltende Schuhdeodorants oder Fußsprays; Schuheinlagen, welche z.B. mit trocknenden Agentien wie Zinkoxid, Soda, Aktivkohle und/oder Zedernholz ausgerüstet sind; die Sterilisation von Schuhwerk mittels UV-Strahlen; das Tragen von Naturwollsocken, z.B. Merinowollsocken und/oder der Gebrauch von bestimmten Nahrungsergänzungsmitteln, z.B. Zink.

Diese bekannten Mittel und Verfahren sind aber aus verschiedenen Gründen nicht für eine rasch wirkende, langanhaltende und/oder dauerhafte Behandlung und/oder Prophylaxe von Körpergeruch geeignet. In den meisten der vorgenannten Fälle ist eine regelmäßige, meist tägliche, Anwendung bzw. Behandlung nötig. In einigen der vorgenannten Fälle wird lediglich der unangenehme Geruch überlagert bzw. maskiert, ohne jedoch dessen Ursache, wenigstens für einen längeren Zeitraum, zu beseitigen

Es besteht demnach ein Bedürfnis für ein Mittel zur rasch und langanhaltend wirkenden Behandlung von Körpergeruch bei Menschen, vorzugsweise von Körpergeruch, welcher von der Hautoberfläche ausgeht. Vorzugsweise soll ein vorgenanntes Mittel auch keine Antibiotika-Resistenzen verursachen oder begünstigen und gut verträglich sein.

Tyrothricin (CAS RN: 1404-88-2; vgl. auch Definition und Beschreibung im Europäischen Arzneibuch, z.B. gemäß Eintrag 01/2005:1662) ist ein Gemisch verschiedener antibakteriell und teils antimykotisch wirksamer linearer und cyclischer Polypeptide aus den Gruppen der Gramicidine und Tyrocidine; daneben kommen in kleinen Mengen weitere strukturverwandte Polypeptide vor. Tyrothricin kann aus der Fermentationsbrühe von "*Brevibacillus brevis Dubos*" gewonnen werden. Es wird den Polypeptid-Antibiotika zugeordnet, denen unter anderem auch Actinomycin, Bacitracin und die Polymyxine angehören. Seiner antibakteriellen Wirkungsweise nach schädigt Tyrothricin die Zellmembran verschiedener Mikroorganismen irreversibel. Es ähnelt darin den antimikrobiellen Peptiden (auch bezeichnet als "host defense peptides"), wie sie aus Eukaryoten bekannt sind, z. B. Defensin und Cathelicidin. Trotz des jahrzehntelangen Einsatzes von Tyrothricin sind bisher keine Resistenzentwicklungen wie bei anderen Antibiotika bekannt geworden.

Tyrothricin wird bereits als lokal wirksames Antibiotikum in Form von Halstabletten bei Halsentzündungen und -schmerzen mit Schluckbeschwerden, bei Rachen- und Kehlkopfentzündungen sowie bei Entzündungen der Mundschleimhaut und des Zahnfleisches eingesetzt.

Ebenfalls bereits bekannt ist die Verwendung von Tyrothricin zur lindernden Behandlung von kleinflächigen, oberflächlichen, wenig nässenden Wunden der Haut mit bakterieller Superinfektion wie z. B. Riss-, Kratz- oder Schürfwunden.

Ein Überblick über bekannte Verwendungen von Tyrothricin findet sich z.B. bei C. Lang et al. in Pharmazie 71 (2016) 299-305.

Im Dokument DE 2316597 wird eine dermatologische Zubereitung beschrieben, welche eine verstärkte Penetration verschiedener pharmakologisch aktiver Stoffe, darunter Tyrothricin als antimikrobielles Mittel, beispielsweise zur oberflächlichen Behandlung von Verbrennungen, in und durch das Gewebe bzw. die Haut ermöglicht. Die beschriebene Zubereitung enthält Zuckerester oder -ether in Kombination mit einem Sulfoxid oder einem Phosphinoxid.

Im Dokument DE 36 04 865 A1 wird ein Haut-Kosmetikum beschrieben, welches u.a. Tyrothricin enthält und zur Behandlung bei superinfektiösen Dermatosen geeignet ist.

Im Dokument EP 0 355 536 A2 wird ein flexibler, hydrophiler Gelfilm sowie ein Verfahren zu dessen Herstellung und Verwendung beschrieben. Tyrothricin wird als eines der Anwendungsbeispiele für bukkale Applikationsformen des Gelfilms angeführt.

Im Dokument WO 99/061011 wird die Verwendung von Tyrothricin zur Behandlung gewisser Virusinfektionen, u.a. bei der Behandlung von Dellwarzen auf der Haut, beschrieben.

Das Deutsche Patent- und Markenamt hat in der Prioritätsanmeldung zu vorliegender Anmeldung folgenden Stand der Technik recherchiert:
US 2007 / 0 292 355 A1 sowie

Knols B.G.J.: On human odor, malaria mosquitoes, and Limburger cheese.. In: The Lancet., Vol. 348, 1996, Nov. 9, 1322.

Das Dokument US 3 624 200 A betrifft die Kontrolle der Transpiration auf der menschlichen Haut mit Scopolaminestern. Darin werden Zusammensetzungen offenbart zur Kontrolle von Schweissgerüchen auf menschlicher Haut durch Scopolamin-Ester oder deren physiologisch akzeptable Säureadditionssalze, welche in topischen Trägern dispergiert sind.

Es war eine primäre Aufgabe der vorliegenden Erfindung, ein therapeutisches oder nichttherapeutisches Mittel zur Anwendung bei der Behandlung und/oder Prophylaxe von Körpergeruch, vorzugsweise von Körpergeruch, welcher von der Hautoberfläche ausgeht, bei Menschen zur Verfügung zu stellen, welches gegenüber dem Stand der Technik zusätzliche Vorteile aufweist.

Eine weitere Aufgabe der Erfindung war es, eine therapeutische oder nicht-therapeutische, vorzugsweise kosmetische, Zusammensetzung bereitzustellen, die geeignet ist zur Anwendung bei der Behandlung und/oder Prophylaxe von Körpergeruch bei Menschen, insbesondere von Körpergeruch, welcher von der Hautoberfläche ausgeht. Eine spezifische weitere Aufgabe der Erfindung war es auch, solche vorgenannten therapeutischen oder nicht-therapeutischen, vorzugsweise kosmetischen, Zusammensetzungen bereitzustellen, welche keinen oder nur einen geringen Eigengeruch aufweisen.

Eine weitere Aufgabe der Erfindung bestand darin, ein Kit zur Verfügung zu stellen, welches geeignet ist zur Anwendung bei der Behandlung und/oder Prophylaxe von Körpergeruch bei Menschen, insbesondere von Körpergeruch, welcher von der Hautoberfläche ausgeht.

Die Erfindung sowie erfindungsgemäß bevorzugte Kombinationen bevorzugter Parameter, Eigenschaften und/oder Bestandteile der vorliegenden Erfindung werden in den beigefügten Ansprüchen näher angegeben und/oder definiert.

Spezielle und/oder bevorzugte Ausführungsformen der Erfindung sowie deren erfindungsgemäße Kombinationen werden nachfolgend genauer beschrieben.

Ausführungsformen, Aspekte, Parameter oder Eigenschaften, welche im Zusammenhang mit der vorliegenden Erfindung für die erfindungsgemäße Verwendung bzw. Anwendung von Tyrothricin beschrieben oder als bevorzugt beschrieben werden, gelten jeweils entsprechend bzw. sinngemäß auch fürdie erfindungsgemäße Zusammensetzung bzw. deren Verwendung oder Anwendung, das erfindungsgemäße Kit bzw. dessen Verwendung.

Es wurde nun gefunden, dass die primäre Aufgabe sowie weitere Aufgaben und/oder Teilaufgaben der vorliegenden Erfindung gelöst werden durch die therapeutische, vorzugsweise pharmazeutische, oder nicht-therapeutische, vorzugsweise kosmetische, Verwendung von Tyrothricin und/oder einer Tyrothricin umfassenden erfindungsgemäßen Zusammensetzung zur Behandlung und/oder Prophylaxe von Körpergeruch bei Menschen, wie in den beigefügten Ansprüchen angegeben.

Bevorzugt ist eine vorstehend angegebene, erfindungsgemäße Verwendung von Tyrothricin, wobei der Körpergeruch hervorgerufen und/oder gefördert und/oder ausgelöst wird durch bakterielle Besiedlung und/oder bakterielle Stoffwechselprodukte und/oder Schweißbildung und/oder wobei der Körpergeruch mit einer oder mehreren der vorgenannten Erscheinungen einhergeht.

Weiter ist eine erfindungsgemäße Verwendung oder eine bevorzugte erfindungsgemäße Verwendung bevorzugt, wobei der Körpergeruch ausgeht vom Fuß. Intertriginöse Bereiche umfassen allgemein schlecht durchlüftete Bereiche der Hautoberfläche in der Leistengegend (inguinal) und/oder unter dem Brustbereich (submammär), vorzugsweise unter dem weiblichen Brustbereich, und/oder Zwischenräume der Fußzehen.

Ebenfalls bevorzugt ist eine erfindungsgemäße oder vorstehend als bevorzugt angegebene erfindungsgemäße Verwendung, wobei der Körpergeruch ausgeht vom Fuß, einschließlich der intertriginösen Bereiche des Fußes.

Sofern der Körpergeruch ausgeht vom Fuß, ist die erfindungsgemäße Verwendung von Tyrothricin besonders bevorzugt bei übermäßiger Schweißbildung, vorzugsweise bei Vorliegen übermäßiger Schweißbildung, die einem Erscheinungsbild zuzuordnen ist, welches ausgewählt ist aus der Gruppe bestehend aus Hyperhidrose, vorzugsweise primäre fokale Hyperhidrose, *Hyperhidrosis plantaris, Hyperhidrosis axillaris* und/oder *Hyperhidrosis pedis; Keratoma sulcatum* (auch bezeichnet als "*plantar pitting*" oder "*pitted keratolysis*", siehe oben) und Bromhidrose, vorzugsweise apokrine Bromhidrose, ekkrine Bromhidrose und/oder keratogene ekkrine Bromhidrose; sowie Mischformen der vorgenannten Erscheinungsbilder.

Ferner ist auch bevorzugt eine erfindungsgemäße oder vorstehend als bevorzugt angegebene erfindungsgemäße Verwendung, wobei die Applikation lokal, oberflächlich erfolgt und/oder wobei die Applikation auf der wundfreien Haut erfolgt.

"Wundfreie Haut" bedeutet im Rahmen der vorliegenden Erfindung, dass die Haut vorzugsweise unverletzt ist und insbesondere keine oberflächlichen Wunden, wie z. B. Riss-, Kratz- oder Schürfwunden, aufweist. Warzen, Verhornungen und/oder Nagelmykosen fallen dagegen im Rahmen der vorliegenden Erfindung nicht unter den Begriff "wundfreie Haut". Entsprechend und sinngemäß bedeutet "wundfreie Schleimhaut" allgemein, dass die Schleimhaut vorzugsweise unverletzt ist. Klarstellend sei betont, dass kleinere oberflächliche Wunden der Haut wie kleinere Riss-, Kratz- oder Schürfwunden aus rein medizinischer Sicht unbedenklich sind für die therapeutische oder nicht-therapeutische Verwendung von Tyrothricin zur Behandlung und/oder Prophylaxe von Körpergeruch bei Menschen und keinen Hinderungsgrund bzw. keine Kontraindikation für eine vorgenannte erfindungsgemäße Verwendung bzw. Anwendung darstellen.

Eine "lokale, oberflächliche Verwendung bzw. Anwendung" bedeutet im Rahmen der vorliegenden Erfindung, dass vorzugsweise der Wirkstoff Tyrothricin nicht perkutan und/oder nicht systemisch und/oder nicht transdermal, d.h. nicht durch Aufnahme über die Haut in den Organismus, beispielsweise nicht in die Blut- oder Lymphbahn, wirkt, also nicht durch Resorption oder Absorption von Tyrothricin durch die Haut und/oderdurch die Schleimhaut hindurch. Eine erfindungsgemäße lokale und/oder oberflächliche Anwendung oder Verwendung soll aber das Eindringen von Tyrothricin in die oberste Hautschicht bzw. Schicht der Epidermis (*Stratum corneum*) hinein, und gegebenenfalls auch in darunterliegende Hautschichten, mit einschließen.

In eigenen Untersuchungen wurde gefunden, dass bei einer mindestens einmal täglichen lokalen, oberflächlichen Anwendung einer Tyrothricin enthaltenden Zusammensetzung über einen Zeitraum ("Behandlungszeitraum") im Bereich von 1 bis 7 Tagen, etwa im Bereich von 2 bis 7 Tagen wie im Bereich von 3 bis 7 Tagen und in der Regel im Bereich von 5 bis 6 Tagen, ein als unangenehm empfundener Körpergeruch signifikant vermindert und überraschender Weise oft sogar dauerhaft beseitigt wird ("Anwendungserfolg").

Weiter wurde in eigenen Untersuchungen gefunden, dass besagte signifikante Verminderung oder sogar Beseitigung des als unangenehm empfundenen Körpergeruchs bei einer signifikanten Anzahl der untersuchten Fälle üblicher Weise nach Beendigung der Anwendung der Tyrothricin enthaltenden Zusammensetzung noch über einen längeren Zeitraum ("Erfolgszeitraum") im Bereich von 7 bis 14 Tagen, in vielen Fällen sogar im Bereich von 7 bis 21 Tagen, in einer signifikanten Anzahl von Fällen auch im Bereich von 7 bis 28 Tagen und in einigen Fällen noch im Bereich von 7 bis 30 Tagen, anhielt. Eine Wiederholung der Anwendung mit einer Tyrothricin enthaltenden Zusammensetzung war somit erst nach Ablauf des Erfolgszeitraums, in den meisten Fällen nach Ablauf von 7 Tagen nach Abschluss des oder eines vorhergehenden Behandlungszeitraumes, in der Mehrzahl der Fälle nach Ablauf von 14 Tagen nach Abschluss des oder eines vorhergehenden Behandlungszeitraumes, in vielen Fällen nach Ablauf von 21 Tagen nach Abschluss des oder eines vorhergehenden Behandlungszeitraumes und in einigen Fällen nach Ablauf von 28 Tagen nach Abschluss des oder eines vorhergehenden Behandlungszeitraumes, wieder angezeigt. Die vorliegende Erfindung betrifft auch Tyrothricin zur Anwendung bei der Behandlung und/oder Prophylaxe von Körpergeruch bei Menschen, wie in den beigefügten Ansprüchen angegeben. Tyrothricin ist hierbei vorzugsweise Bestandteil einer erfindungsgemäßen Zusammensetzung, wie sie nachfolgend beschrieben wird.

Alle vorstehend für die erfindungsgemäße Verwendung von Tyrothricin angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für Tyrothricin zur vorstehend angegebenen erfindungsgemäßen Anwendung.

Die vorliegende Erfindung offenbart bzw. betrifft auch ein Verfahren zur therapeutischen oder nicht-therapeutischen, vorzugsweise kosmetischen, Behandlung und/oder Prophylaxe von Körpergeruch beim Menschen, wie in den beigefügten Ansprüchen angegeben, wobei Tyrothricin oder eine Tyrothricin enthaltende Zusammensetzung, vorzugsweise eine erfindungsgemäße Zusammensetzung, wie sie nachfolgend beschrieben wird, lokal, oberflächlich auf die, vorzugsweise wundfreie, Haut und/oder die, vorzugsweise wundfreie, Schleimhaut appliziert wird. Vorzugsweise erfolgt die vorgenannte Applikation auf die, bevorzugt wundfreie, Haut. Vorzugsweise erfolgt die vorgenannte Applikation oder bevorzugte Applikation in einer therapeutisch bzw. nicht-therapeutisch, vorzugsweise kosmetisch, wirksamen Dosis.

Alle vorstehend für die erfindungsgemäße Verwendung von Tyrothricin angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für das vorstehend angegebene erfindungsgemäße Verfahren.

Ein vorstehend angegebenes erfindungsgemäßes Verfahren und/oder eine erfindungsgemäße Verwendung und/oder eine erfindungsgemäße Anwendung von Tyrothricin und/oder einer Tyrothricin enthaltenden, vorzugsweise erfindungsgemäßen, Zusammensetzung (vorzugsweise wie sie nachfolgend beschrieben wird), jeweils zur Behandlung und/oder Prophylaxe von Körpergeruch bei Menschen, ist grundsätzlich geeignet zur täglichen Anwendung, vorzugsweise zur ein- oder zweimaligen täglichen Anwendung, auch über längere Zeiträume.

Aufgrund der vorteilhaften Eigenschaften des erfindungsgemäßen Verfahrens und/oder der erfindungsgemäßen Verwendung und/oder der erfindungsgemäßen Anwendung von Tyrothricin und/oder einer Tyrothricin enthaltenden, vorzugsweise erfindungsgemäßen, Zusammensetzung - insbesondere aufgrund der rasch und langanhaltend wirkenden Behandlung bzw. Prophylaxe von Körpergeruch bei Menschen - ist in vielen Fällen nur eine weniger häufige Anwendung erforderlich.

Bevorzugt ist ein vorstehend angegebenes erfindungsgemäßes Verfahren und/oder eine erfindungsgemäße Verwendung und/oder eine erfindungsgemäße Anwendung von Tyrothricin und/oder einer Tyrothricin enthaltenden, vorzugsweise erfindungsgemäßen, Zusammensetzung (vorzugsweise wie sie nachfolgend beschrieben wird), wie in den beigefügten Ansprüchen angegeben, jeweils zur Behandlung und/oder Prophylaxe von Körpergeruch bei Menschen,
wobei Tyrothricin und/oder eine, vorzugsweise erfindungsgemäße, Tyrothricin enthaltende Zusammensetzung (vorzugsweise wie sie nachfolgend beschrieben wird)
für einen Behandlungszeitraum oder einen ersten Behandlungszeitraum, vorzugsweise im Bereich von 1 bis 7 Tagen, bevorzugt im Bereich von 2 bis 7 Tagen, besonders bevorzugt im Bereich von 3 bis 7 Tagen und ganz besonders bevorzugt im Bereich von 5 bis 6 Tagen,
mindestens einmal täglich, vorzugsweise zweimal täglich, besonders bevorzugt zweimal täglich, je einmal morgens und abends, lokal, oberflächlich appliziert wird,
   wobei vorzugsweise der Behandlungszeitraum nicht häufiger wiederholt wird als alle 7 Tage, besonders bevorzugt nicht häufiger als alle 14 Tage,
   und/oder
   wobei vorzugsweise die Applikation auf die wundfreie Haut und/oder die wundfreie Schleimhaut erfolgt.

In einer bevorzugten Ausführungsform des vorstehend angegebenen bevorzugten erfindungsgemäßen Verfahrens und/oder der vorstehend angegebenen bevorzugten erfindungsgemäßen Verwendung und/oder der vorstehend angegebenen bevorzugten erfindungsgemäßen Anwendung wird Tyrothricin und/oder eine, vorzugsweise erfindungsgemäße, Tyrothricin enthaltende Zusammensetzung nach einem ersten Behandlungszeitraum (wie vorstehend definiert oder wie vorstehend als bevorzugt definiert),

für einen zweiten oder weiteren Behandlungszeitraum ("Rezidiv-Prophylaxe"), vorzugsweise im Bereich von 1 bis 4 Tagen, bevorzugt im Bereich von 2 bis 3 Tagen,
mindestens einmal täglich lokal, oberflächlich appliziert, vorzugsweise zweimal täglich, besonders bevorzugt zweimal täglich, je einmal morgens und abends,
wobei vorzugsweise der zweite oder weitere Behandlungszeitraum nicht häufiger wiederholt wird als alle 30 Tage, besonders bevorzugt nicht häufiger als alle 21 Tage, ganz besonders bevorzugt nicht häufiger als alle 14 Tage, jeweils gerechnet vom Ende des ersten bzw. vorhergehenden Behandlungszeitraums an,
und/oder
wobei vorzugsweise die Applikation auf die wundfreie Haut und/oder die wundfreie Schleimhaut erfolgt.

Vorzugsweise kann im Rahmen der vorstehend angegebenen erfindungsgemäßen Verwendung und/oder Anwendung und/oder des vorstehend angegebenen erfindungsgemäßen Verfahrens Tyrothricin oder eine Tyrothricin enthaltende Zusammensetzung lokal, oberflächlich in üblichen Darreichungsformen appliziert werden. Für die Applikation, vorzugsweise auf die wundfreie, Hautoberfläche eignen sich beispielsweise an sich bekannte Cremes, Gele, Lotionen, Puder, Pulver, Pulversprays, Roll-on-Zubereitungen, Salben, Schäume, Sprays, Stifte und Tinkturen. Für die Applikation einer Tyrothricin enthaltenden Zusammensetzung auf die, vorzugsweise wundfreie, Schleimhaut eignen sich - im Falle der Mundschleimhaut - beispielsweise an sich bekannte Bonbons, Pastillen, Lutschtabletten, Sublingualtabletten, Buccaltabletten, Dragees bzw. Spül- oder Gurgellösungen. Für die Applikation einer Tyrothricin enthaltenden Zusammensetzung auf die, vorzugsweise wundfreie, Schleimhaut eignen sich - im Falle der Vaginalschleimhaut - beispielsweise an sich bekannte Cremes, Gele, Lotionen, Puder, Pulversprays, Salben, Schäume, Sprays, Tinkturen, Spüllösungen oder Suppositorien.

Weiter betrifft die vorliegende Erfindung auch eine therapeutische, vorzugsweise pharmazeutische, oder nicht-therapeutische, vorzugsweise kosmetische, Zusammensetzung, enthaltend Tyrothricin, vorzugsweise in einer antimikrobiell wirksamen Menge, und mindestens ein Keratolytikum, vorzugsweise in einer keratolytisch wirksamen Menge, vorzugsweise zur Anwendung bei der Behandlung und/oder Prophylaxe von Körpergeruch bei Menschen. Bevorzugte erfindungsgemäße Zusammensetzungen sind geruchsneutral. Im Gegensatz dazu weisen viele Zusammensetzungen des Standes der Technik zur Behandlung und/oder Prophylaxe von Körpergeruch bei Menschen - etwa Deodorants oder Anti-transpirantien - einen mehr oder weniger starken Eigengeruch auf.

Die erfindungsgemäße Zusammensetzung ist zum Einsatz im Rahmen der erfindungsgemäßen Verwendung von Tyrothricin bzw. im Rahmen des erfindungsgemäßen Aspektes "Tyrothricin zur Anwendung bei der Behandlung und/oder Prophylaxe von Körpergeruch bei Menschen" geeignet und dafür vorgesehen, vorzugsweise bei der Behandlung und/oder Prophylaxe von Körpergeruch bei Menschen, wobei der Körpergeruch ausgeht von der Hautoberfläche, vorzugsweise vom Fuß und/oder der Achselhöhle und/oder einem intertriginösen Bereich.

Ohne Gewähr der Richtigkeit wird angenommen, dass durch die Wirkung des Keratolytikums mindestens die oberste Hautschicht, vorzugsweise die oberste Schicht der Epidermis (*Stratum corneum*) angelöst oder aufgelöst wird, so dass zum Einen weniger oder keine Hautreste mehr für eine Zersetzung durch Mikroorganismen wie Bakterien zur Verfügung stehen und dass zum Anderen das lokale Einwirken des Wirkstoffes Tyrothricin auf die Hautoberfläche bzw. die Hautpartie, von welcher ein als unangenehm empfundener Körpergeruch ausgeht, erleichtert, begünstigt und/oder erst ermöglicht wird.

In der vorgenannten erfindungsgemäßen Zusammensetzung ist das Keratolytikum vorzugsweise ausgewählt aus der Gruppe bestehend aus α-Hydroxysäuren, vorzugsweise Glycolsäure, Mandelsäure und/oder Milchsäure; Acitretin; Adapalen; Allantoin; Aluminiumoxid; Azelainsäure; Benzoylperoxid; Harnstoff; Isotretinoin; Monochloressigsäure; Motretinid; Retinoiden; Salicylsäure; Schieferölen; Selendisulfid; Tazaroten; Teeren; Tretinoin und Mischungen der vorstehenden Keratolytika. Erfindungsgemäß ist Harnstoff besonders bevorzugt als Keratolytikum.

Bevorzugt ist eine erfindungsgemäße oder vorstehend oder nachfolgend als bevorzugt angegebene erfindungsgemäße Zusammensetzung, worin Tyrothricin in einer Menge in einem Bereich von 0,01 bis 0,5 Gew.-%, vorzugsweise in einem Bereich von 0,05 bis 0,2 Gew.-%, bezogen auf die Gesamtmasse der Zusammensetzung, enthalten ist
und/oder (vorzugsweise "und")
worin Harnstoff in einer Menge in einem Bereich von 0,5 bis 20,0 Gew.-%, vorzugsweise in einem Bereich von 1,0 bis 15,0 Gew.-%, besonders bevorzugt in einem Bereich von 3,0 bis 12,0 Gew.-%, bezogen auf die Gesamtmasse der Zusammensetzung, enthalten ist.

Bevorzugt ist eine erfindungsgemäße oder vorstehend oder nachfolgend als bevorzugt angegebene erfindungsgemäße Zusammensetzung, weiter enthaltend ein oder mehrere Polyethylenglykole (nachfolgend auch als "PEG" abgekürzt) und/oder Propylenglykol. Solche erfindungsgemäßen Zusammensetzungen können vorteilhafterweise - insbesondere bei Verzicht auf sonstige Bestandteile - geruchsneutral (geruchsfrei) sein; eine solche Zusammensetzung ist besonders bevorzugt.

Bevorzugte Polyethylenglykole für den Einsatz in erfindungsgemäßen oder bevorzugten erfindungsgemäßen Zusammensetzungen sind ausgewählt aus der Gruppe der Polyethylenglykole mit einer mittleren relativen Molekülmasse im Bereich von 200 bis 12000, vorzugsweise im Bereich von 200 bis 8000, am meisten bevorzugt im Bereich von 200 bis 6000, vorzugsweise bestimmt mittels Größenausschluss-Chromatographie. Besonders bevorzugte Typen von Polyethylenglykolen sind ausgewählt aus der Gruppe bestehend aus PEG 300, PEG 400, PEG 600, PEG 1500, PEG 2000, PEG 3000 und PEG 4000, wobei die Ziffern in für den Fachmann üblicher Weise jeweils die mittleren relativen Molekülmassen angeben. Es kann ein Polyethylenglykol-Typ einer bestimmten mittleren relativen Molekülmasse, z.B. "PEG 300" oder "PEG 1500", alleine eingesetzt werden oder es können vorteilhaft Mischungen aus zwei oder mehr Polyethylenglykol-Typen jeweils einer bestimmten mittleren relativen Molekülmasse eingesetzt werden, z.B. "PEG 300" in Mischung mit "PEG 1500". Durch die Auswahl von Polyethylenglykol-Typen geeigneter mittlerer relativer Molekülmassen oder von Mischungen von zwei oder mehr Polyethylenglykol-Typen jeweils einer bestimmten mittleren relativen Molekülmasse können erfindungsgemäße Zusammensetzungen verschiedener Konsistenzen bzw. Viskositäten hergestellt und an den gewünschten Verwendungszweck (etwa als Salbe oder als Befüllung für einen Deoroller) angepasst hergestellt werden.

Propylenglykol (1,2-Dihydroxypropan) umfasst für die Zwecke der vorliegenden Erfindung alle Isomere dieser Verbindung, sowohl die optisch aktiven Verbindungen ((R)- und (S)-1,2-Dihydroxypropan) als auch deren Mischungen, insbesondere das Racemat.

Bevorzugt ist eine erfindungsgemäße oder vorstehend oder nachfolgend als bevorzugt angegebene erfindungsgemäße Zusammensetzung, welche Polyethylenglykole und/oder Propylenglykol (wie vorstehend beschrieben jeweils einzeln oder als Mischungen mehrere Polyethylenglykol-Typen miteinander und/oder mit Propylenglykol) in einer Gesamtmenge (Polythylenglykol(e) plus Propylenglykol) im Bereich von 15 bis 90 Gew.-%, vorzugsweise im Bereich von 30 bis 90 Gew.-% enthält, besonders bevorzugt im Bereich von 40 bis 90 Gew.-%, bezogen auf. die Gesamtmasse der Zusammensetzung.

Die vorstehend beschriebenen bevorzugten erfindungsgemäßen Zusammensetzungen, welche ein oder mehrere Polyethylenglykole und/oder Propylenglykol enthalten, weisen eine Anzahl von Vorteilen auf:
So können vorgenannte erfindungsgemäße Zusammensetzungen, welche eine Gesamtmenge von 15 Gew.-% oder mehr, bezogen auf das das Gesamtgewicht (bzw. die Gesamtmasse) der Zusammensetzung, an ein oder mehreren Polyethylenglykolen und/oder Propylenglykol enthalten, antibakterielle bzw. konservierende Wirkung aufweisen, so dass zusätzliche Konservierungsmittel in der Regel nicht notwendig sind bzw. den Zusammensetzungen nicht zugesetzt werden müssen.

Erfindungsgemäße Zusammensetzungen öliger Konsistenz ("Öle") werden zur Erhöhung der Stabilität vorzugsweise in braunen Glasflaschen abgefüllt und gelagert, erfindungsgemäße Zusammensetzungen in Form einer Salbe werden vorzugsweise in einer beschichteten, vorzugsweise PVC-freien, Aluminiumtube abgefüllt und gelagert.

Weiterhin weisen Polyethylenglykole und Propylenglykol eine sehr gute Verträglichkeit auf.

Es hat sich außerdem in eigenen Versuchen gezeigt, dass eine möglicherweise zu befürchtende (gegebenenfalls langsam eintretende) Zersetzung von Harnstoff und dadurch ansteigende pH-Werte in der Zusammensetzung, welche zu Beeinträchtigungen des Wirkstoffes Tyrothricin führen könnten, in erfindungsgemäßen Zusammensetzungen, welche Polyethylenglykole und/oder Propylenglykol enthielten, nicht auftraten bzw. nicht beobachtet wurden. Im Gegenteil waren solche vorgenannten bevorzugten erfindungsgemäßen Zusammensetzungen auch über längere Zeiträume stabil.

Ein weiterer Vorteil des Einsatzes von Propylenglykol im Sinne der vorliegenden Erfindung ist eine penetrationsfördernde Wirkung, wodurch die vorstehend beschriebene vorteilhafte keratolytische Wirkung von Harnstoff unterstützt bzw. verstärkt werden kann.

Besonders bevorzugt ist daher eine erfindungsgemäße oder vorstehend oder nachfolgend als bevorzugt angegebene erfindungsgemäße Zusammensetzung, welche neben Tyrothricin und Harnstoff nur Polyethylenglykole und/oder Propylenglykol enthält.

In vielen Fällen ist auch eine erfindungsgemäße oder vorstehend oder nachfolgend als bevorzugt angegebene erfindungsgemäße Zusammensetzung bevorzugt, welche zusätzlich ein Benetzungsmittel enthält, vorzugsweise ausgewählt aus der Gruppe bestehend aus Polydimethylsiloxanen, Polyethylenoxiden, Polysorbaten sowie deren Mischungen. Besonders bevorzugt als Benetzungsmittel ist in diesem Zusammenhang Polydimethylsiloxan (PDMS), auch bekannt unter der Bezeichnung Dimeticon (INN), CAS RN 9006-65-9.

Ebenfalls in vielen Fällen bevorzugt ist eine erfindungsgemäße oder vorstehend oder nachfolgend als bevorzugt angegebene erfindungsgemäße Zusammensetzung, welche zusätzlich einen hautverträglichen pH-Regulator enthält, vorzugsweise ein Puffersystem, besonders bevorzugt ein Puffersystem, welches zur Einstellung bzw. Pufferung eines pH-Wertes im Bereich von 7,1 bis 8,5, vorzugsweise im Bereich von 7,5 bis 8,0, geeignet ist, beispielsweise an sich bekannte Phosphatpuffer, Tris(hydroxymethyl)-aminomethan-Puffer (auch bezeichnet als Trometamol) oder Borat-Puffer. Eine erfindungsgemäße Zusammensetzung, welche als Keratolytikum Harnstoff enthält, besitzt vorzugsweise einen pH-Wert, der nicht höher ist als 8,0 und vorzugsweise durch ein Puffersystem stabilisiert ist.

Ebenfalls bevorzugt ist eine erfindungsgemäße oder vorstehend oder nachfolgend als bevorzugt angegebene erfindungsgemäße Zusammensetzung, welche zusätzlich ein Vergällungsmittel enthält, vorzugsweise ein Denatonium-Derivat, besonders bevorzugt ausgewählt aus der Gruppe bestehend aus Denatoniumbenzoat und Denatoniumsaccharinat.

Vorzugsweise ist die erfindungsgemäße oder vorstehend oder nachfolgend als bevorzugt angegebene erfindungsgemäße Zusammensetzung eine topische Zusammensetzung, besonders bevorzugt in einer Form ausgewählt aus der Gruppe bestehend aus Creme, Gel, Lotion, Puder, Pulver, Pulverspray, Öl, Roll-on-Zubereitung, Salbe, Schaum, Spray, Stift und Tinktur, besonders bevorzugt aus der Gruppe bestehend aus Creme, Gel, Lotion, Puder, Pulver, Pulverspray, Öl, Roll-on-Zubereitung, Salbe und Spray.

Zur erfindungsgemäß bevorzugten Verwendung bzw. Anwendung am Fuß, einschließlich zur Verwendung zum Zweck einer Rezidiv-Prophylaxe, ist bevorzugt eine topische erfindungsgemäße Zusammensetzung in einer Form ausgewählt aus der Gruppe bestehend aus Creme, Puder, Pulver, Pulverspray, Salbe und Spray. Besonders bevorzugt in diesem Zusammenhang ist eine topische erfindungsgemäße Zusammensetzung in Form einer Creme, einer Salbe oder eines Pulvers. Am meisten bevorzugt in diesem Zusammenhang ist eine topische erfindungsgemäße Zusammensetzung in Form einer Creme oder einer Salbe.

Zur erfindungsgemäß bevorzugten Verwendung bzw. Anwendung in bzw. unter der Achselhöhle, einschließlich zur Verwendung zum Zweck einer Rezidiv-Prophylaxe, ist bevorzugt eine topische erfindungsgemäße Zusammensetzung in einer Form ausgewählt aus der Gruppe bestehend aus Gel, Lotion, Puder, Öl, Roll-on-Zubereitung, Schaum, Spray und Stift. Besonders bevorzugt in diesem Zusammenhang ist eine topische erfindungsgemäße Zusammensetzung in Form einer Creme, einer Salbe, eines Öls oder einer Roll-on-Zubereitung.

Zur Verwendung bzw. Anwendung an bzw. auf der Schleimhaut ist bevorzugt eine topische Zusammensetzung in einer Form ausgewählt aus der Gruppe bestehend aus Creme, Gel, Lotion, Puder, Pulver, Pulverspray, Salbe, Schaum, Spray, Stift und Tinktur. Besonders bevorzugt in diesem Zusammenhang ist eine topische Zusammensetzung in Form einer Creme, eines Gels, einer Salbe, eines Schaums oder eines Sprays.

Die Erfindung betrifft auch die Verwendung einer erfindungsgemäßen oder vorstehend als bevorzugt angegebenen erfindungsgemäßen Zusammensetzung und/oder einer erfindungsgemäßen oder vorstehend als bevorzugt angegebenen erfindungsgemäßen topischen Zusammensetzung, jeweils zur Behandlung und/oder Prophylaxe von Körpergeruch bei Menschen, vorzugsweise bei der Behandlung und/oder Prophylaxe von Körpergeruch bei Menschen, wobei der Körpergeruch ausgeht von der Hautoberfläche, vorzugsweise vom Fuß und/oder der Achselhöhle und/oder einem intertriginösen Bereich.

Alle vorstehend für die erfindungsgemäße Verwendung von Tyrothricin angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für die vorstehend angegebene Verwendung einer erfindungsgemäßen oder bevorzugten erfindungsgemäßen Zusammensetzung und/oder einer topischen Zusammensetzung, jeweils zur Behandlung und/oder Prophylaxe von Körpergeruch bei Menschen.

Ebenfalls betrifft die Erfindung eine erfindungsgemäße oder vorstehend als bevorzugt angegebene erfindungsgemäße Zusammensetzung und/oder eine erfindungsgemäße oder vorstehend als bevorzugt angegebene erfindungsgemäße topische Zusammensetzung, jeweils zur Anwendung bei der Behandlung und/oder Prophylaxe von Körpergeruch bei Menschen

Alle vorstehend für die erfindungsgemäße Verwendung von Tyrothricin angegebenen erfindungsgemäßen Ausgestaltungen und Kombinationen, einschließlich der jeweils als bevorzugt angegebenen Ausgestaltungen und Kombinationen, gelten ohne Einschränkung und gegebenenfalls sinngemäß auch für die vorstehend angegebene Zusammensetzung und/oder topische Zusammensetzung, jeweils zur Anwendung bei der Behandlung und/oder Prophylaxe von Körpergeruch bei Menschen.

Die Verwendung bzw. Anwendung der erfindungsgemäßen oder einer vorstehend als bevorzugt angegebenen erfindungsgemäßen Zusammensetzung enthaltend Tyrothricin und ein Keratolytikum, vorzugsweise Harnstoff, ist bevorzugt an der Hautoberfläche, bevorzugt an Stellen, an denen die Haut bzw. Hornhaut schlecht durchlüftet ist und/oder an Stellen, an denen die Haut bzw. Hornhaut aufgequollen und/oder ganz oder teilweise mazeriert ist, Läsionen aufweist (v.a. in Fällen von "*Keratoma sulcatum*", "*pitted keratolysis*" oder "*plantar pitting*", s.o.) und/oder Symptome von Bromhidrose, insbesondere Bromhyperhidrose, apokriner Bromhidrose, ekkriner Bromhidrose und/oder keratogener ekkriner Bromhidrose aufweist. Besonders bevorzugt ist die Verwendung bzw. Anwendung der erfindungsgemäßen oder einer bevorzugten erfindungsgemäßen Zusammensetzung daher an der Hautoberfläche des Fußes, insbesondere der Fußsohle und/oder den Zehenzwischenräumen, der Handflächen und/oder in intertriginösen Bereichen, am meisten bevorzugt an der Hautoberfläche des Fußes, insbesondere der Fußsohle und/oder den Zehenzwischenräumen.

Die vorliegende Erfindung betrifft auch ein Kit, enthaltend die räumlich getrennten Bestandteile
a) eine zur topischen Verabreichung geeignete Zusammensetzung enthaltend Tyrothricin in einer antimikrobiell wirksamen Menge und
b) mindestens eine zur topischen Verabreichung geeignete Zusammensetzung enthaltend jeweils mindestens ein Keratolytikum in einer keratolytisch wirksamen Menge.

Eine "zur topischen Verabreichung geeignete Zusammensetzung" ist vorzugsweise ausgewählt aus der Gruppe bestehend aus Creme, Gel, Lotion, Puder, Pulver, Pulverspray, Roll-on-Zubereitung, Salbe, Schaum, Spray, Stift und Tinktur, besonders bevorzugt aus der Gruppe bestehend aus Creme, Gel, Lotion, Puder, Pulver, Pulverspray, Roll-on-Zubereitung, Salbe und Spray. Im Falle des erfindungsgemäßen Kits sind vom Begriff "zur topischen Verabreichung geeignete Zusammensetzung" auch Vormischungen und Vorprodukte umfasst, die vor einer bestimmungsgemäßen Anwendung bzw. Applikation erst in eine gebrauchsfertige Form bzw. in eine gebrauchsfertige Zusammensetzung überführt werden müssen.

In einer Ausführungsform betrifft die Erfindung auch einen vorstehend angegebenen Kit, zur Anwendung bei der Behandlung und/oder Prophylaxe von Körpergeruch bei Menschen und/oder die Verwendung eines vorstehend angegebenen, erfindungsgemäßen Kits zur Behandlung und/oder Prophylaxe von Körpergeruch bei Menschen.

In einer weiteren Ausführungsform betrifft die Erfindung auch einen Kit zur vorstehend angegebenen Anwendung bzw. Verwendung bei der Behandlung und/oder Prophylaxe von Körpergeruch bei Menschen, wobei die Anwendung das vorzugsweise abwechselnde Auftragen der Komponenten a) und b) auf die wundfreie Haut über einen Behandlungszeitraum umfasst, welcher ausreicht, um den mit Schweißbildung einhergehenden Körpergeruch mindestens für einen Erfolgszeitraum zu vermindern, vorzugsweise mindestens für einen Erfolgszeitraum zu beseitigen. Die Begriffe Behandlungseitraum bzw. Erfolgszeitraum haben dabei im Rahmen der vorliegenden Erfindung vorzugsweise die gleiche Bedeutung wie bereits vorstehend (im Zusammenhang mit der Beschreibung eines Anwendungserfolges) angegeben.

Der vorliegende Text offenbart weiterhin einen Fußbekleidungsartikel, vorzugsweise Schuheinlage, Einlegesohle, Strumpf, Socke oder Füßling, enthaltend eine antimikrobiell wirksame Menge von Tyrothricin.

Vorzugsweise wird unter allen erfindungsgemäßen Aspekten (Verwendung, Anwendung, Zusammensetzung, Verwendung/Anwendung der Zusammensetzung; Kit, Verwendung des Kits) neben Tyrothricin kein weiterer antibakterieller (Antibiotikum) und/oder antiseptischer Wirkstoff verwendet, wobei jedoch ein erfindungsgemäß einsetzbares Keratolytikum, vorzugsweise Harnstoff, im Rahmen der vorliegenden Erfindung nicht als vorzugsweise zu vermeidender antibakterieller und/oder antiseptischer Wirkstoff anzusehen ist. Mit der Verwendung von Tyrothricin als einzigem antibakteriellem und/oder antiseptischem Wirkstoff ist u.a. der Vorteil verbunden, dass eine erfindungsgemäße Behandlung von Körpergeruch nur ein sehr geringes Risiko für die Entwicklung bzw. Entstehung resistenter Keime mit sich bringt.

### Beispiele:

Die nachfolgend angegebenen Beispiele sollen die Erfindung näher beschreiben, ohne ihren Umfang zu beschränken.

### Beispiel 1: Herstellung einer erfindungsgemäßen Zusammensetzung (Salbe), enthaltend Tyrothricin und ein Keratolytikum

Die nachfolgend in Tabelle 1 angegebenen Inhaltsstoffe bzw. Bestandteile wurden zusammengefügt und auf an sich bekannte Weise miteinander zu einer homogen erscheinenden Salbe vermischt, die geruchsneutral erschien.

**Tabelle 1: Zusammensetzung einer erfindungsgemäßen Salbe**

| **Inhaltsstoff** | **Menge [g]** |
|---|---|
| Tyrothricin | 0,1 |
| Harnstoff (als Keratolytikum) | 10,0 |
| PEG 300 | 43,2 |
| PEG 1500 | 46,7 |

Die in Beispiel 1 hergestellte, erfindungsgemäße Zusammensetzung (Salbe) eignet sich besonders zum Auftragen auf die von Körpergeruch betroffene Haut des Fußes, insbesondere der Fußsohlen.

### Beispiel 2: Herstellung einer erfindungsgemäßen Zusammensetzung (Öl), enthaltend Tyrothricin und ein Keratolytikum

Die nachfolgend in Tabelle 2 angegebenen Inhaltsstoffe bzw. Bestandteile wurden zusammengefügt und auf an sich bekannte Weise miteinander zu einem homogen erscheinenden Öl vermischt, das farblos und geruchsneutral erschien.

**Tabelle 2: Zusammensetzung eines erfindungsgemäßen Öls**

| **Inhaltsstoff** | **Menge [g]** |
|---|---|
| Tyrothricin | 0,1 |
| Harnstoff (als Keratolytikum) | 10,0 |
| PEG 300 | 89,9 |

Die in Beispiel 2 hergestellte, erfindungsgemäße Zusammensetzung (Öl) eignet sich beispielsweise zum Befüllen eines Deorollers oder zum direkten Auftragen auf von Körpergeruch betroffene Stellen der Haut, etwa an den Fußsohlen oder axillär bzw. in intertriginösen Bereichen.

### Beispiel 3: Herstellung einer erfindungsgemäßen Zusammensetzung (Öl), enthaltend Tyrothricin und ein Keratolytikum

Die nachfolgend in Tabelle 3 angegebenen Inhaltsstoffe bzw. Bestandteile wurden zusammengefügt und auf an sich bekannte Weise miteinander zu einem homogen erscheinenden Öl vermischt, das farblos und geruchsneutral erschien.

**Tabelle 3: Zusammensetzung eines weiteren erfindungsgemäßen Öls**

| **Inhaltsstoff** | **Menge [g]** |
|---|---|
| Tyrothricin | 0,1 |
| Harnstoff (als Keratolytikum) | 10,0 |
| Propylenglykol (PH. EUR. 8.0) | 89,9 |

Die in Beispiel 3 hergestellte, erfindungsgemäße Zusammensetzung (Öl) eignet sich beispielsweise zum Befüllen eines Deorollers oder zum direkten Auftragen auf von Körpergeruch betroffene Stellen der Haut, etwa an den Fußsohlen oder axillär bzw. in intertriginösen Bereichen.

### Beispiel 4: Testreihe zur Überprüfung der Wirksamkeit von Tyrothricin bei der Behandlung von Körpergeruch an Testpersonen

Es wurde eine Testreihe mit 22 freiwilligen Personen ("Testpersonen"; 14 männliche, 8 weibliche) durchgeführt, welche zu Beginn der Testreihe unter als unangenehm empfundenem Körpergeruch litten, der von der Hautoberfläche des Fußes ausging ("Fußgeruch").

Zur Beurteilung der Stärke des Fußgeruches der Testpersonen wurde vom Versuchsleiter eine subjektive Skala der Geruchsempfindung angewendet, wonach die Testpersonen in die folgenden, in Tabelle 4 angegebenen Gruppen eingeteilt wurden:

**Tabelle 4: Einteilung der Testpersonen nach Stärke des Fußgeruches**

| **Skalenwert** | **Stärke des Fußgeruches** | **Anzahl Testpersonen** |
|---|---|---|
| 0 | Neutral | Keine Behandlung nötig |
| 1 | Kaum merklich | Ausschlusskriterium |
| 2 | Merklich | 1 |
| 3 | Deutlich wahrzunehmen | 7 |
| 4 | Stark | 8 |
| 5 | Sehr stark | 6 |

Einschlusskriterien für die Teilnahme an der Testreihe:
- Fußgeruch von mindestens der Stärke 2 auf der subjektiven Skala nach Tabelle 4
- Intakte, nicht krankhaft veränderte Haut des Fußes bzw. der Fußsohlen (keine oberflächlichen Wunden oder Verletzungen).

Ausschlusskriterien für die Teilnahme an der Testreihe:
- Krankhafte Veränderungen der Haut des Fußes (Fußrücken, Fußsohle, Zehenzwischenräume) wie offene Wunden, allergische oder idiopathische Dermatosen, Ulzera etc.; kein Ausschlusskriterium waren Verhornungen, Warzen oder Nagelmykosen;

### Durchführung der Testreihe:

Den Testpersonen wurde über einen Zeitraum von jeweils 6 Tagen ("Behandlungszeitraum") einmal täglich eine Tyrothricin enthaltende Zusammensetzung ("Testsalbe", enthaltend 0,1 % Tyrothricin und entsprechend der Zusammensetzung des Beispiels 1) vollflächig auf die Leistenhaut und ca. 5 mm über den Grenzbereich zur Cutis des rechten Fußes aufgetragen (linker Fuß diente zur Kontrolle), unter Einbeziehung der Zehen und Zehenzwischenräume. Die Fußnägel wurden von der Anwendung ausgenommen und wurden vor Beginn der Testreihe möglichst weit zurückgeschnitten. Nach dem Auftragen der Testsalbe wurden die Füße der Testpersonen für mindestens 12 Stunden nicht gewaschen.

Die Testpersonen sollten während der Dauer der Testreihe und für einen Anschlusszeitraum nach Beendigung der Testreihe (30 Tage) ihre Körperpflegegewohnheiten nicht verändern, jedoch etwaige individuelle Maßnahmen oder Mittel zur Behandlung des Fußgeruches (andere als die erfindungsgemäße Behandlung) für die Dauer der Testreihe und/oder des Anschlusszeitraumes absetzen. Das Tragen von Schuhwerk war erlaubt, Barfußlaufen aber möglichst zu unterlassen.

An jedem der 6 Behandlungstage wurde vor Applikation der Testsalbe jeweils von dem Versuchsleiter die Stärke des Fußgeruches (gemäß Skala in Tabelle 2) beurteilt und notiert. Vor erneutem Auftragen der Testsalbe wurde jeweils sichergestellt, dass durch die oder während der Behandlung keine unerwünschten oder krankhaften Hautveränderungen eingetreten waren.

### Testergebnisse:

Unmittelbar nach Abschluss des Behandlungszeitraumes konnten folgende Ergebnisse erzielt werden (Skalenwerte der Stärke des Fußgeruches gemäß Tabelle 4):
Bei der Testperson mit Fußgeruch des Skalenwertes 2 war bereits am 2. Behandlungstag kaum noch als unangenehm empfundener Fußgeruch wahrzunehmen.

Bei den Testpersonen mit Fußgeruch der Skalenwerte 3 bis 5 war ab dem 3. Behandlungstag jeweils ein signifikanter Rückgang des (gegebenenfalls) als unangenehm empfundenen Fußgeruches um jeweils 2 bis 3 Skalenwerte festzustellen.

Ab dem 5. Behandlungstag war bei keiner der Testpersonen mehr ein stärkerer Fußgeruch wahrzunehmen, als dem Skalenwert 2 entsprechend. Bei 15 Testpersonen wurde die Stärke des Fußgeruches unmittelbar nach Ende des Behandlungszeitraumes mit Skalenwerten von 0 oder 1 bewertet.

Es traten keine unerwünschten Nebenwirkungen (wie Hautirritationen oder unerwünschte Geruchsveränderungen) bei irgendeiner der Testpersonen auf.

Die Behandlung mit der Testsalbe war bei allen Testpersonen erfolgreich, es wurden keine Therapieversager identifiziert.

Nach Abschluss des Behandlungszeitraumes wurden die Testpersonen noch über einen Anschlusszeitraum von 28 Tagen wöchentlich über die Andauer des Behandlungserfolges befragt. Das Ergebnis dieser wöchentlichen Befragungen war, dass bei 66 % der Testpersonen auch 14 Tage nach Abschluss der Behandlung das positive Behandlungsergebnis noch andauerte. Bei 50 % der Testpersonen dauerte das positive Behandlungsergebnis auch 21 Tage nach Abschluss der Behandlung noch an.

Als Ergebnis der vorgenannten Testreihe kann somit festgehalten werden, dass die Behandlung von als unangenehm empfundenem Körpergeruch mit Tyrothricin rasch eintritt, über einen längeren Zeitraum (in der Regel ≥ 14 Tage) anhält, mit hoher Wahrscheinlichkeit erfolgreich (es wurden keine Therapieversager identifiziert) und gut verträglich (es wurden keine Nebenwirkungen festgestellt) ist.

## Patentansprüche

1. Nicht-therapeutische Verwendung von Tyrothricin zur Behandlung und/oder Prophylaxe von Körpergeruch bei Menschen, wobei der Körpergeruch ausgeht vom Fuß, einschließlich der intertriginösen Bereiche des Fußes.

2. Tyrothricin zur Verwendung bei der Behandlung und/oder Prophylaxe von Körpergeruch bei Menschen, wobei der Körpergeruch ausgeht vom Fuß, einschließlich der intertriginösen Bereiche des Fußes.

3. Verwendung nach Anspruch 1 oder 2, wobei der Körpergeruch hervorgerufen und/oder gefördert und/oder ausgelöst wird durch bakterielle Besiedlung und/ oder bakterielle Stoffwechselprodukte und/oder Schweißbildung und/oder wobei der Körpergeruch mit einer oder mehreren der vorgenannten Erscheinungen einhergeht.

4. Verwendung nach einem der vorstehenden Ansprüche, wobei die Applikation lokal, oberflächlich erfolgt
und/oder
wobei die Applikation auf der wundfreien Haut erfolgt.

5. Therapeutische, vorzugsweise pharmazeutische, oder nicht-therapeutische, vorzugsweise kosmetische, Zusammensetzung, enthaltend Tyrothricin, vorzugsweise in einer antimikrobiell wirksamen Menge, und mindestens ein Keratolytikum, vorzugsweise in einer keratolytisch wirksamen Menge, vorzugsweise zur Behandlung und/oder Prophylaxe von Körpergeruch bei Menschen.

6. Zusammensetzung nach Anspruch 5, worin das Keratolytikum ausgewählt ist aus der Gruppe bestehend aus α-Hydroxysäuren, vorzugsweise Glycolsäure, Mandelsäure und/oder Milchsäure; Acitretin; Adapalen; Allantoin; Aluminiumoxid; Azelainsäure; Benzoylperoxid; Harnstoff; Isotretinoin; Monochloressigsäure; Motretinid; Retinoiden; Salicylsäure; Schieferölen; Selendisulfid; Tazaroten; Teeren; Tretinoin und Mischungen der vorstehenden Keratolytika.

7. Zusammensetzung nach Anspruch 5 oder 6, worin das Keratolytikum Harnstoff ist.

8. Zusammensetzung nach einem der Ansprüche 5 bis 7,
worin Tyrothricin in einer Menge in einem Bereich von 0,01 bis 0,5 Gew.-%, vorzugsweise in einem Bereich von 0,05 bis 0,2 Gew.-%, bezogen auf die Gesamtmasse der Zusammensetzung, enthalten ist
und/oder
worin Harnstoff in einer Menge in einem Bereich von 0,5 bis 20,0 Gew.-%, vorzugsweise in einem Bereich von 1,0 bis 15,0 Gew.-%, besonders bevorzugt in einem Bereich von 3,0 bis 12,0 Gew.-%, bezogen auf die Gesamtmasse der Zusammensetzung, enthalten ist.

9. Zusammensetzung nach einem der Ansprüche 5 bis 8, vorzugsweise nach Anspruch 7 oder 8, weiter enthaltend ein oder mehrere Polyethylenglykole und/oder Propylenglykol.

10. Topische Zusammensetzung nach einem der Ansprüche 5 bis 9, vorzugsweise in einer Form ausgewählt aus der Gruppe bestehend aus Creme, Gel, Lotion, Puder, Pulver, Pulverspray, Öl, Roll-on-Zubereitung, Salbe, Schaum, Spray, Stift und Tinktur, besonders bevorzugt aus der Gruppe bestehend aus Creme, Gel, Lotion, Puder, Pulver, Pulverspray, Öl, Roll-on-Zubereitung, Salbe und Spray.

11. Zusammensetzung nach einem der Ansprüche 5 bis 9 und/oder topische Zusammensetzung nach Anspruch 10, jeweils zur Verwendung bei der Behandlung und/oder Prophylaxe von Körpergeruch bei Menschen, wobei vorzugsweise der Körpergeruch ausgeht vom Fuß und/oder von der Achselhöhle und/oder einem intertriginösen Bereich.

12. Kit, enthaltend die räumlich getrennten Bestandteile
a) eine zur topischen Verabreichung geeignete Zusammensetzung enthaltend Tyrothricin in einer antimikrobiell wirksamen Menge und
b) mindestens eine zur topischen Verabreichung geeignete Zusammensetzung enthaltend jeweils mindestens ein Keratolytikum in einer keratolytisch wirksamen Menge.

13. Verfahren zur nicht-therapeutischen, vorzugsweise kosmetischen, Behandlung und/oder Prophylaxe von Körpergeruch beim Menschen, wobei Tyrothricin oder eine Tyrothricin enthaltende Zusammensetzung, lokal, oberflächlich auf die, vorzugsweise wundfreie, Haut des Fußes appliziert wird.

## Claims

1. Nontherapeutic use of tyrothricin for treatment and/or prophylaxis of human body odour, wherein the body odour emanates from the foot, including the intertriginous regions of the foot.

2. Tyrothricin for use in the treatment and/or prophylaxis of human body odour, wherein the body odour emanates from the foot, including the intertriginous regions of the foot.

3. Use according to Claim 1 or 2, wherein the body odour is caused and/or promoted and/or triggered by bacterial colonization and/or bacterial metabolism products and/or perspiration and/or wherein the body odour is associated with one or more of the aforementioned phenomena.

4. Use according to any of the preceding claims, wherein the application is local and superficial
and/or
wherein the application is to the wound-free skin.

5. Therapeutic, preferably pharmaceutical, or nontherapeutic, preferably cosmetic, composition comprising tyrothricin, preferably in an antimicrobially effective amount, and at least one keratolytic, preferably in a keratolytically effective amount, preferably for treatment and/or prophylaxis of human body odour.

6. Composition according to Claim 5, in which the keratolytic is selected from the group consisting of α-hydroxy acids, preferably glycolic acid, mandelic acid and/or lactic acid; acitretin; adapalene; allantoin; aluminium oxide; azelaic acid; benzoyl peroxide; urea; isotretinoin; monochloroacetic acid; motretinide; retinoids; salicylic acid; shale oils; selenium disulfide; tazarotene; tars; tretinoin and mixtures of the aforementioned keratolytics.

7. Composition according to Claim 5 or 6, in which the keratolytic is urea.

8. Composition according to any of Claims 5 to 7,
in which tyrothricin is present in an amount within a range from 0.01% to 0.5% by weight, preferably within a range from 0.05% to 0.2% by weight, based on the total mass of the composition,
and/or
in which urea is present in an amount within a range from 0.5% to 20.0% by weight, preferably within a range from 1.0% to 15.0% by weight, more preferably within a range from 3.0% to 12.0% by weight, based on the total mass of the composition.

9. Composition according to any of Claims 5 to 8, preferably according to Claim 7 or 8, further comprising one or more polyethylene glycols and/or propylene glycol.

10. Topical composition according to any of Claims 5 to 9, preferably in a form selected from the group consisting of cream, gel, lotion, powder, powder spray, oil, roll-on formulation, ointment, foam, spray, stick and tincture, more preferably from the group consisting of cream, gel, lotion, powder, powder spray, oil, roll-on formulation, ointment and spray.

11. Composition according to any of Claims 5 to 9 and/or topical composition according to Claim 10, in each case for use in the treatment and/or prophylaxis of human body odour, wherein the body odour preferably emanates from the foot and/or from the armpit and/or an intertriginous region.

12. Kit comprising the following spatially separated constituents:
a) a composition suitable for topical administration and comprising tyrothricin in an antimicrobially effective amount and
b) at least one composition suitable for topical administration and comprising at least one keratolytic in each case in a keratolytically effective amount.

13. Method of nontherapeutic, preferably cosmetic, treatment and/or prophylaxis of human body odour, wherein tyrothricin or a tyrothricin-containing composition is applied locally and superficially to the preferably wound-free skin of the foot.

## Revendications

1. Utilisation non thérapeutique de tyrothricine pour le traitement et/ou la prophylaxie d'odeurs corporelles chez l'homme, dans laquelle les odeurs corporelles partent du pied, y compris des zones intertrigineuses du pied.

2. Tyrothricine destinée à être utilisée lors du traitement et/ou de la prophylaxie d'odeurs corporelles chez l'homme, dans laquelle les odeurs corporelles partent du pied, y compris des zones intertrigineuses du pied.

3. Utilisation selon la revendication 1 ou 2, dans laquelle les odeurs corporelles sont provoquées et/ou favorisées et/ou déclenchées par une colonisation bactérienne et/ou des métabolites d'origine bactérienne et/ou par la formation de sueur et/ou dans laquelle les odeurs corporelles vont de pair avec une ou plusieurs des manifestations susmentionnées.

4. Utilisation selon l'une quelconque des revendications précédentes, dans laquelle l'application a lieu de manière locale, en surface,
et/ou
dans laquelle l'application a lieu sur la peau sans plaie.

5. Composition thérapeutique, de préférence pharmaceutique, ou non thérapeutique, de préférence cosmétique, contenant de la tyrothricine, de préférence en une quantité présentant une efficacité en matière d'action antimicrobienne, et au moins un agent kératolytique, de préférence en une quantité présentant une efficacité en matière d'action kératolytique, de préférence pour le traitement et/ou la prophylaxie d'odeurs corporelles chez l'homme.

6. Composition selon la revendication 5, dans laquelle l'agent kératolytique est choisi parmi le groupe constitué de alpha-hydroxyacides, de préférence acide glycolique, acide mandélique et/ou acide lactique ; acitrétine ; adapalène ; allantoïne ; oxyde d'aluminium ; acide azélaïque ; peroxyde de benzoyle ; urée ; isotrétinoïne ; acide monochloroacétique ; motrétinide ; rétinoïdes ; acide salicylique ; huiles de schiste ; disulfure de sélénium ; tazarotène ; goudrons ; trétinoïne et des mélanges des agents kérolytiques ci-avant.

7. Composition selon la revendication 5 ou 6, dans laquelle l'agent kératolytique est de l'urée.

8. Composition selon l'une quelconque des revendications 5 à 7,
dans laquelle de la tyrothricine est contenue en une quantité dans la plage de 0,01 à 0,5 % en poids, de préférence dans une plage de 0,05 à 0,2 % en poids, par rapport à la masse totale de la composition
et/ou
dans laquelle de l'urée est contenue en une quantité dans une plage de 0,5 à 20,0 % en poids, de préférence dans une plage de 1,0 à 15,0 % en poids, de manière particulièrement préférée dans une plage de 3,0 à 12,0 % en poids, par rapport à la masse totale de la composition.

9. Composition selon l'une quelconque des revendications 5 à 8, de préférence selon la revendication 7 ou 8, contenant par ailleurs un ou plusieurs polyéthylèneglycols et/ou du propylèneglycol.

10. Composition topique selon l'une quelconque des revendications 5 à 9, de préférence sous une forme choisie parmi le groupe constitué de crème, gel, lotion, poudre très fine, poudre, pulvérisateur de poudre, huile, préparation sous forme de bille, onguent, mousse, spray, bâton et teinture, de manière particulièrement préférée parmi le groupe constitué de crème, gel, lotion, poudre très fine, poudre, pulvérisateur de poudre, huile, préparation sous forme de bille, onguent et spray.

11. Composition selon l'une quelconque des revendications 5 à 9 et/ou composition topique selon la revendication 10, destinées respectivement à être utilisées lors du traitement et/ou de la prophylaxie d'odeurs corporelles chez l'homme, dans laquelle ou lesquelles de préférence les odeurs corporelles partent du pied et/ou des aisselles et/ou d'une zone intertrigineuse.

12. Ensemble contenant les constituants séparés spatialement
a) une composition adaptée pour l'administration topique, contenant de la tyrothricine en une quantité présentant une efficacité en matière d'action antimicrobienne, et
b) au moins une composition adaptée pour l'administration topique, contenant respectivement au moins un agent kératolytique en une quantité présentant une efficacité en matière d'action kératolytique.

13. Procédé de traitement et/ou de prophylaxie non thérapeutique, de préférence cosmétique, d'odeurs corporelles chez l'homme, dans lequel de la tyrothricine ou une composition contenant de la tyrothricine est appliquée localement, en surface sur la peau, de préférence sans plaie, du pied.
